# EUROPEAN PATENT SPECIFICATION

(11) **EP 2 303 372 B1**
(45) Date of publication and mention of the grant of the patent: **15.05.2013**
(21) Application number: 09789852.2
(22) Date of filing: 19.06.2009
(51) Int. Cl.: A61M 16/00, G06F 3/048

(54) **USER INTERFACE FOR BREATHING ASSISTANCE SYSTEM**
BENUTZEROBERFLÄCHE FÜR EIN ATEMHILFSSYSTEM
INTERFACE UTILISATEUR POUR UN SYSTÈME D'ASSISTANCE RESPIRATOIRE

(30) Priority: 08.07.2008 US 168981
(43) Date of publication of application: 06.04.2011
(73) Proprietor: Nellcor Puritan Bennett LLC, North Haven, Connecticut 06473 (US)
(72) Inventor: DESFOSSEZ, Benjamin, F-74300 Cluses (FR); MICHEL, Patrick, F-54000 Nancy (FR); NADJAFIZADEH, Hossein, F-54600 Villers-les-nancy (FR)
(74) Representative: Beyer, Andreas
(86) International application number: PCT/US2009/047893
(87) International publication number: WO 2010/005763

(56) References cited:
- WO-A-00/45880
- WO-A-98/41271
- WO-A-02/058619
- CAIRO J M ET AL: "Mosby's respiratory care equipment , passage" MOSBY'S RESPIRATORY CARE EQUIPMENT, ST. LOUIS, MO : MOSBY INC, US, 1 January 2004 (2004-01-01), pages 477-494, XP002427922 ISBN: 978-0-323-02215-6

## Description

### TECHNICAL FIELD

The present disclosure relates generally to breathing assistance systems, and particularly to a user interface for a breathing assistance system.

### BACKGROUND

A breathing assistance system typically delivers pressurized breathing gas to a patient via tubing called a "patient interface" or a "breathing circuit." The breathing gas typically includes air and/or one or more gasses (e.g., oxygen mixed with the air). The breathing gas delivered to the patient from the breathing assistance system may be humidified and/or heated in the breathing circuit before being delivered to the patient. The breathing assistance system typically increases the pressure in the breathing circuit so that the breathing gas is pushed into the lungs for inspiration, and reduces the pressure in the breathing circuit so that gases in the lungs can be expired and vented to the atmosphere. Typically, one or more breathing assistance system parameters may be determined and/or adjusted prior to and/or during operation, e.g., the mode of ventilation (e.g., CMV (controlled mandatory ventilation), SIMV (synchronized intermittent mandatory ventilation), CPAP (constant positive airway pressure), or bi-level CPAP); the patient's tidal volume (the volume of gas inspired with each breath); the respiratory rate (the number of breaths per minute (BPM)); and/or the O₂ concentration, flow rate, airway pressure, and/or minute volume (the volume inspired and expired in one minute) of breathing gas delivered to the patient.

A user interface may permit a user (e.g., a patient or caregiver) to set, control, and/or adjust the various breathing assistance system parameters. For example, a user interface may include a display, buttons, touch screen, and/or other input means allowing a user to input data and/or set breathing assistance parameters. However, due to the operational complexity of some breathing assistance systems, many users have difficulty using traditional user interfaces.

WO 02/058619 A2 discloses a ventilator interface for a clinician comprising a touch screen. The interface displays a row of publicly available ventilator control settings, including a middle column comprising a list of parameter identifiers. By touching a row, a control slider appears on the screen, which the clinician can operate to change target values for various parameters. The most recently detected value for each parameter is displayed to the right of each parameter identifier. If the current parameter is inactive, an OFF indicator is located to the left of the parameter identifier.

Cairo, J. M., et. Al: «Mosby's Respiratory Care Equipment», St. Louis, Mosby Inc., US, 1 January 2004 (2004-01-01), pages 477-496, XP002427922 ISBN: 978-0-323-02215-6 discloses a ventilator having a control screen with two operating knobs. By rotating one of the knobs, a user can scroll through various options, selecting a desired option which the user would like to change. Only parameters which can be changed in the current mode are displayed. When the user has selected a desired parameter, rotating a knob causes adjustment of a numerical value associated with the selected parameter.

WO 98/41271 A discloses a ventilation control system comprising a user-friendly interface for the display of patient data and ventilator status, as well as for entering values for ventilation setting to be used to control the ventilator and for setting and displaying appropriate alarms settings and patient data. Various pre-assigned buttons are located below a bottom portion of the display, and may be operated to perform predetermined functions. If a button for adjusting contrast or brightness to the display, or for adjusting a system volume is operated, a rotating knob may be rotated to increase or decrease these parameters. Additionally, on screen touch buttons may be operated to adjust various parameters of the ventilation apparatus.

### SUMMARY

In accordance with the teachings of the present disclosure, disadvantages and problems associated with traditional user interfaces used for breathing assistance systems have been substantially reduced or eliminated.

A user interface according to claim 1 is provided. The user interface may include a plurality of user-activated buttons configured to receive user input and a display communicatively coupled to the plurality of buttons. The display may include a plurality of function indicators, each function indicator associated with a corresponding one of the user-activated buttons and configured to display a different graphical function representations in different situations, the different graphical function representations indicating different functions of the corresponding user-activated button. The display may also include a modification indicator configured to display different graphical modification representations in different situations to indicate whether or not a selected parameter value may be modified using one or more of the user-activated buttons.

A breathing assistance system according to claim 8 is provided. The breathing assistance system may include a gas delivery system operable to supply breathable gas, a patient interface configured to interface with a patient for delivering breathable gas to the patient, a connection system configured to communicate breathable gas supplied by the gas delivery system to the patient interface for delivery to the patient, a user interface, and a control module communicatively coupled to the user interface and configured to regulate the delivery of breathable gas to the patient based at least on user input received by the user interface. The user interface may include a plurality of user-activated buttons configured to receive user input and a display communicatively coupled to the plurality of buttons. The display may include a plurality of function indicators, each function indicator associated with a corresponding one of the user-activated buttons and configured to display a different graphical function representations in different situations, the different graphical function representations indicating different functions of the corresponding user-activated button. The display may also include a modification indicator configured to display different graphical modification representations in different situations to indicate whether or not a selected parameter value may be modified using one or more of the user-activated buttons.

A method according to claim 9 is provided. A method for displaying user interface functionality information to a user is provided. The method may include providing a plurality of user-activated buttons configured to receive user input. The method may also include displaying a plurality of function indicators, each function indicator associated with a corresponding one of the user-activated buttons and configured to display a different graphical function representations in different situations, the different graphical function representations indicating different functions of the corresponding user-activated button. The method may further include displaying a modification indicator configured to display different graphical modification representations in different situations to indicate whether or not a selected parameter value may be modified using one or more of the user-activated buttons.

Other technical advantages will be apparent to those of ordinary skill in the art in view of the following specification, claims, and drawings.

### BRIEF DESCRIPTION OF THE DRAWINGS

A more complete understanding of the present embodiments may be acquired by referring to the following description taken in conjunction with the accompanying drawings, in which like reference numbers indicate like features, and wherein:
FIGURE 1 illustrates a block diagram of an example breathing assistance system, in accordance with certain embodiments of the disclosure;
FIGURE 2 illustrates an example user interface for a breathing assistance system, in accordance with certain embodiments of the disclosure; and
FIGURES 3A-3F illustrate various example user interface screens displayed to a user interface, in accordance with certain embodiments of the present disclosure.

### DETAILED DESCRIPTION

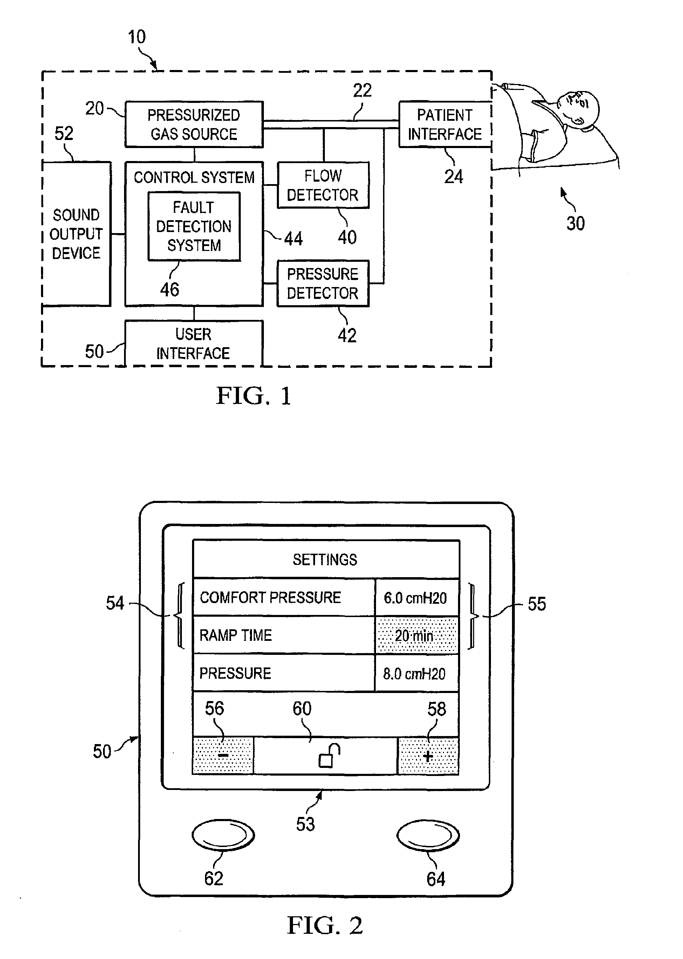

Embodiments of the disclosure may be understood by reference to FIGURES 1 through 3F, wherein like numbers are used to indicate like and corresponding parts.

FIGURE 1 illustrates a block diagram of an example breathing assistance system 10, in accordance with certain embodiments of the present disclosure. In general, a user interface may allow a user to view, set, control, and/or adjust parameters associated with the operation of the breathing assistance system.

As used herein, the terms "gas" and/or "breathable gas" may refer to any one or more gases and/or vaporized substances suitable to be delivered to and/or from a patient via one or more breathing orifices (e.g., the nose and/or mouth), such as air, nitrogen, oxygen, any other component of air, CO₂, vaporized water, vaporized medicines, and/or any combination of two or more of the above, for example.

As used herein, the term "patient" may refer to any person or animal that may receive breathing assistance from system 10, regardless of the medical status, official patient status, physical location, or any other characteristic of the person. Thus, for example, patients may include persons under official medical care (e.g., hospital patients), persons not under official medical care, persons receiving care at a medical care facility, persons receiving home care, etc.

As shown in FIGURE 1, breathing assistance system 10 may include a gas flow source 20, a connection system 22, a patient interface 24, a flow detector 40, a pressure detector 42, a control system 44, and a user interface 50. Breathing assistance system 10 may comprise any device, apparatus, or system for delivering breathable gas to a patient, e.g., a ventilator, a respirator, a CPAP device, or a BiPAP device. Gas flow source 20 may comprise any system or device suitable for generating and/or delivering pressurized gas (e.g., air and/or oxygen or one or more other supplemental gasses) toward a patient 30, including without limitation, a blower, a compressor, a piston-based device, one or more pressurized gas tanks, one or more gas lines (e.g., from a wall or other source), or any combination thereof.

Connection system 22 may include any system or device suitable for delivering pressurized gas generated by gas flow source 20 towards patient 30, e.g., a connection system and/or other conduits and connection devices. Patient interface 24 may include any system or device suitable for further delivering pressurized gas delivered by connection system 22 to patient 30, e.g., a nasal or face mask, nasal pillows, and/or a tube (e.g., an endotracheal tube, a tracheostomy tube and/or other tracheal tube).

Flow detector 40 may generally be operable to detect the flow rate of gas flowing through one or more conduits of system 10, e.g., the flow rate produced by gas flow source 20 or the flow rate of gas delivered to patient 30. Flow detector 40 may include any number of sensors operable to detect flow rate of a gas and/or any device operable to convert a detected flow rate into electrical signals or otherwise sense flow rate. Flow detector 40 may be placed at any suitable location and in any suitable orientation for sensing flow rate of a gas within breathing assistance system 10. For example, flow detector 40 may be placed within connection system 22, or near gas flow source 20, an air intake port, and/or an air outlet point.

Pressure detector 42 may generally be operable to detect a pressure of gas within one or more conduits of breathing assistance system 10 by gas flow source 20 and/or the pressure of gas delivered to patient 30. Pressure detector 42 may include any number of sensors operable to detect gas pressure and/or any suitable device operable to convert a detected pressure into electrical signals or otherwise sense pressure. Pressure detector 42 may be placed at any suitable location and in any suitable orientation for sensing gas pressure within breathing assistance system 10. For example, pressure detector 42 may be placed within connection system 22, or near gas flow source 20, an air intake point, and/or an air outlet port.

User interface 50 may include any suitable device or devices allowing a user to interface with breathing assistance system 10, e.g., to input desired patient data that may be communicated to control system 44 to control the operation of gas flow source 20 and/or other components of breathing assistance system 10. For example, user interface 50 may allow a user to input (e.g., via buttons, touch screen, and/or other tactile means) one or more of the following patient data: the age, weight, tidal volume capacity, respiratory rate, inhale sensitivity, exhale sensitivity, and/or other characteristics of patient 30, leak settings, rise time, alarm settings, delay, ramp, starting pressure, inhalation:exhalation (I:E) ratio, flow rate, pressure, a selected ventilation program, and/or any other parameter regarding patient 30 and/or the operation of breathing assistance system 10. User interface 50 may also include a display device that may communicate information to a user regarding patient 30 and/or the operation of breathing assistance system device 10.

Control system 44 may generally be operable to process various inputs, e.g., input from user interface 50, ventilation programs stored in memory, and/or feedback from flow detector 40, pressure detector 42, or other variables sensed or otherwise detected by other sensors associated with breathing assistance system 10, and to regulate the operation of gas flow source 20 or other components of breathing assistance system 10 based on such various inputs. Control system 44 may include any suitable system or device for controlling the operation of breathing assistance system 10, including, e.g., a microcontroller, a digital signal processor (DSP), an application specific integrated controller (ASIC), electrically-programmable read-only memory (EPROM), or a field-programmable gate array (FPGA). In some embodiments, control system 44 may include software and/or other executable code for analyzing input signals received from user interface 50 and/or feedback from flow detector 40, pressure detector 42, or other variables sensed or otherwise detected by other sensors associated with breathing assistance system 10 to generate control signals for regulating the operation of breathing assistance system 10. Such software may include any suitable algorithms, logic and/or instructions for processing signals in breathing assistance system 10, and may be stored in any suitable data storage media.

In some embodiments, control system 44 may control the operation of gas flow source 20. For example, where gas flow source 20 comprises a motorized blower, control system 44 may control the operation (e.g., the motor speed and on/off control) of the blower. In addition, control system 44 may generate sound signals to be broadcast by breathing assistance system 10, such as user feedback (e.g., instructions or other words) or other sounds regarding the operation of breathing assistance system 10. For example, control system 44 may monitor the operation of breathing assistance system 10 and, when appropriate, generate alarm signals (e.g., a siren, buzzer, or words) to be broadcast by a sound output device 52. In the same or alternative embodiments, control system 44 may communicate user feedback to user interface 50 for display to a user (e.g., via a display device), and/or receive input from a user via user interface 50, as described in further detail below.

Thus, control system 44 may provide, without limitation, any or all of the following functions: (a) controlling the operation of gas flow source 20, (b) monitoring the operation of ventilator 10, (c) generating user feedback signals to be broadcast by sound output device 52, (d) generating output to be displayed to user interface 50, and/or (e) receiving user input from user interface 50.

Sound output device 52 may generally be operable to output sound signals generated by control system 44, for example, user feedback and/or alarms. Sound output device 52 may include a speaker and an audio driver operable to control the speaker. In some embodiments, sound output device 52 may simultaneously broadcast multiple sound signals.

FIGURE 2 illustrates an example user interface 50 for breathing assistance system 10 in accordance with one embodiment of the disclosure. As shown in FIGURE 2, user interface 50 may include a display 53 and user input buttons 62 and 64. Display 50 may comprise any system, device, or apparatus configured to display graphic images and/or alphanumeric characters recognizable to a user, and may include, for example, a liquid crystal display (LCD), a cathode ray tube (CRT), a matrix of light emitting diodes (LEDs), or a matrix of organic light emitting diodes (OLEDs). In some embodiments, display 50 may include an illumination device (e.g., in an LCD, a cold cathode florescent lamp or CCFL) or any other suitable device for providing illumination for a display.

Each of buttons 62 and 64 may be any system, device, or apparatus configured to, when pressed or actuated by a user, communicate signals indicative of a user selection to control system 44 and/or another component of breathing assistance system 10. A user's actuation of button 62 and/or 64 may cause a change of an operational parameter of breathing assistance system 10, a change in operation of breathing assistance system 10, and/or a change in the contents of display 53.

Display 53 may include a listing of parameters 54, parameter values 55 associated with parameters 54, function indicators 56 and 58, and modification indicator 60. Listing of parameters 54 may include one or more parameters related to the patient 30 and/or the operation of breathing assistance system 10. For example, parameters 54 may include a mode of breathing assistance (e.g., CMV (controlled mandatory ventilation), SIMV (synchronized intermittent mandatory ventilation), CPAP (constant positive airway pressure), or bi-level CPAP); the tidal volume of patient 30 (the volume of gas inspired with each breath); the respiratory rate of patient 30 (the number of breaths per minute (BPM)); and/or the O₂ concentration, flow rate, airway pressure, and/or minute volume (the volume inspired and expired in one minute) of breathing gas delivered to the patient 30. Parameter values 55 may include operational values (e.g., settings and/or measured values) associated with the displayed parameters 54.

Function indicators 56 and 58 may provide a dynamic graphical indication of the functionality of buttons 62 and 64. For example, in the screen displayed in FIGURE 2, function indicator 56 displays a minus sign, which may indicate that pressing button 62 associated with function indicator 56 may reduce a selected parameter value 55 (e.g., the parameter value for the ramp time, as shown in FIGURE 2). As another example, in the screen displayed in FIGURE 2, function indicator 58 displays a plus sign, which may indicate that pressing button 64 associated with function indicator 58 may increase a selected parameter value 55 (e.g., the parameter value for the ramp time, as shown in FIGURE 2).

Modification indicator 60 may provide a dynamic graphical indication of whether a selected parameter value 55 may be modified by a user via user interface 50. For example, in the screen displayed in FIGURE 2, modification indicator 60 displays an icon of an unlocked padlock, which may indicate that a user may modify a selected parameter value 55 (e.g., the parameter value for the ramp time, as shown in FIGURE 2). In contrast, if modification indicator 60 displays an icon of a locked padlock, it may indicate that a user may not modify a selected parameter value 55.

Although buttons 62 and 64 are shown as separate from display 53, in certain embodiments, display 53 may include a touch screen wherein buttons 62 and 64 are an integral part of display 53. in addition, buttons 62 and 64 and their respective function indicators may be integral to the same element. For example, button 62 and function indicator 56 may be combined, and such combined element may display a graphical representation of the functionality of that element (e.g., the combined element may display a minus sign to indicate that a selected parameter value 55 may be decreased). Similarly, button 64 and function indicator 58 may be combined, and such combined element may display a graphical representation as to the functionality of that element (e.g., the combined element may display a plus sign to indicate that a selected parameter value 55 may be increased).

In addition, although FIGURE 2 depicts user interface 50 as including buttons 62 and 64, user interface 50 may include any suitable number of buttons each having any suitable functionality. For example, user interface 50 may include a button allowing a user to confirm a selection (e.g., similar to an "Enter" or "Return" key on a computer keyboard) and/or may include buttons allowing a user to scroll through options and/or parameters on display 53.

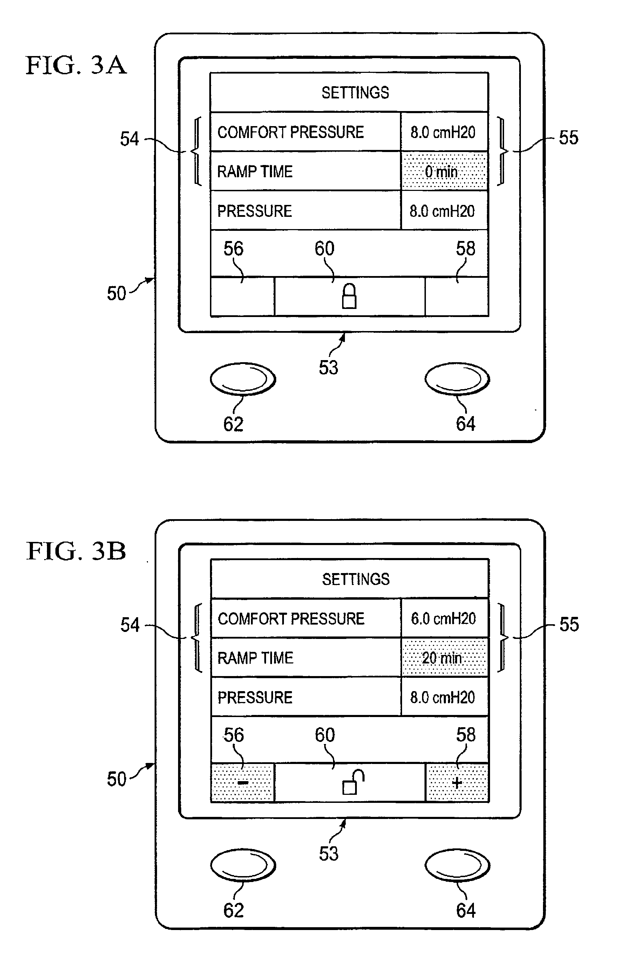

FIGURES 3A-3F illustrate various example user interface screens that may be displayed to display 53 of user interface 50 depending upon the functionality of buttons 62 and 64 and/or the ability to modify a selected parameter value 55, in accordance with the present disclosure. For example, FIGURE 3A depicts an example screen that may be displayed at display 53 in the event that a selected parameter value 55 may not be modified by a user. As shown in FIGURE 3A, the parameters 54 of "comfort pressure" and "pressure" have each been set to 8.0 cm H₂O. Accordingly, a breathing assistance system 10 providing such pressures may not need to ramp the desired comfort pressure to an operating pressure if such values are identical, and the ramp time between the two pressures is zero. Thus, if a user selects the parameter value 55 associated with the parameter "ramp time" (as indicated by the shading in FIGURE 3A), function indicators 56 and 58 may indicate that associated buttons 62 and 64 are not functional to modify the ramp time parameter (e.g., by appearing blank as shown in FIGURE 3A; by a "grayed out" icon; or using an icon indicating the parameter may not be modified, such as an "x," for example). In addition, modification indicator 60 may indicate that the selected parameter "ramp time" may not be modified (e.g., modification indicator 60 may display an icon of a locked padlock as shown in FIGURE 3A, or display appropriate text such as "locked," for example).

As another example, FIGURE 3B depicts an example screen that may be displayed to display 53 in the event that a selected parameter value 55 may be modified by a user with each button 62 and 64 having functionality to modify such parameter value. As shown in FIGURE 3B, the parameters 54 of "comfort pressure" and "pressure" have been set to 6.0 cm H₂O and 8.0 cm H₂O, respectively. Accordingly, a breathing assistance system 10 providing such pressures may ramp from the desired comfort pressure to an operating pressure according to a user setting. Thus, if a user selects the parameter value 55 associated with the parameter "ramp time," function indicators 56 and 58 may indicate that associated buttons 62 and 64 are functional to modify the ramp time parameter. For example, function indicator 56 may display a minus sign indicating that button 62 may decrease the ramp time and/or function indicator 58 may display a plus sign indicating that button 64 may increase the ramp time as shown in FIGURE 3B. In addition, modification indicator 60 may indicate that the selected parameter "ramp time" may be modified (e.g., modification indicator 60 may display an icon of an unlocked padlock as shown in FIGURE 3B; or display appropriate text such as "unlocked," for example).

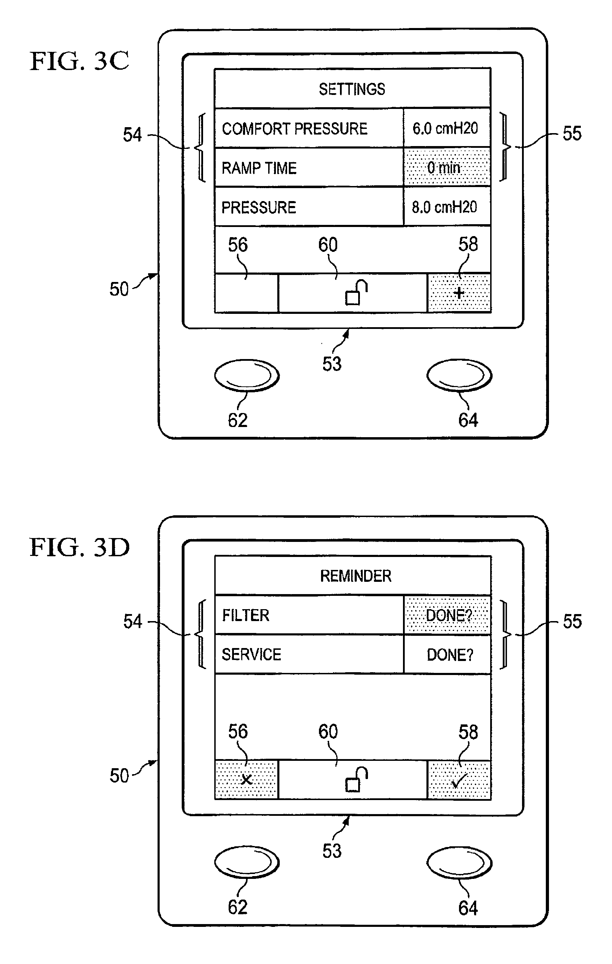

As another example, FIGURE 3C depicts an example screen that may be displayed to display 53 in the event that a selected parameter value 55 may be modified by a user, with only one of buttons 62 and 64 having functionality to modify such parameter value. As shown in FIGURE 3C, the parameters 54 of "comfort pressure" and "pressure" have been set to 6.0 cm H₂O and 8.0 cm H₂O, respectively. Accordingly, a breathing assistance system 10 providing such pressures may ramp from the desired comfort pressure to an operating pressure according to a user setting. However, if the selected parameter value 55 for ramp time is at a minimum or maximum, one of buttons 62 and 64 may be functional to modify the selected parameter value 55, while the other button 62 and 64 may not. Thus, if a user selects the parameter value 55 associated with the parameter "ramp time" and the parameter value 55 is at its minimum value, function indicator 56 may indicate that associated button 62 is not functional to modify the ramp time parameter and/or function indicator 58 may indicate that associated button 64 is functional to modify the ramp time parameter. For example, function indicator 56 may appear blank to indicate that button 62 is not functional to modify the ramp time and/or function indicator 58 may display a plus sign indicating that button 64 may increase the ramp time as shown in FIGURES 3C. Alternatively, function indicator 56 may appear as a "grayed out" icon or an icon indicating that the parameter may not be modified, such as an "x" for example. In addition or alternatively, function indicator 58 may appear as an icon indicated that the parameter may be modified, such as a checkmark, for example. In addition, modification indicator 60 may indicate that the selected parameter "ramp time" may be modified (e.g., modification indicator 60 may display an icon of an unlocked padlock as shown in FIGURE 3C, or display appropriate text such as "unlocked," for example).

As a further example, FIGURE 3D depicts an example screen that may be displayed to display 53 in the event a selected parameter value 55 may be modified by a user and buttons 62 and 64 having functionality to select such parameter value. As shown in FIGURE 3D, a parameter value 55 associated with a filter change is selected. The filter change parameter may have one of two values (e.g., "no" or "false" if the filter has not been changed, "yes" or "true" if the filter has been changed). Thus, if a user selects the filter change parameter value 55, function indicators 56 and 58 may indicate that their associated buttons 62 and 64 may be functional to select a value for such parameter value 55. For example, function indicator 56 may display an "x" to indicate that button 62 is functional to select "no" or "false," and/or function indicator 58 may display a checkmark to indicate that button 64 is functional to select "yes" or "true" as shown in FIGURE 3D. In addition, modification indicator 60 may indicate that the selected parameter value may be modified and/or selected (e.g., modification indicator 60 may display an icon of an unlocked padlock as shown in FIGURE 3D, or display appropriate text such as "unlocked," for example).

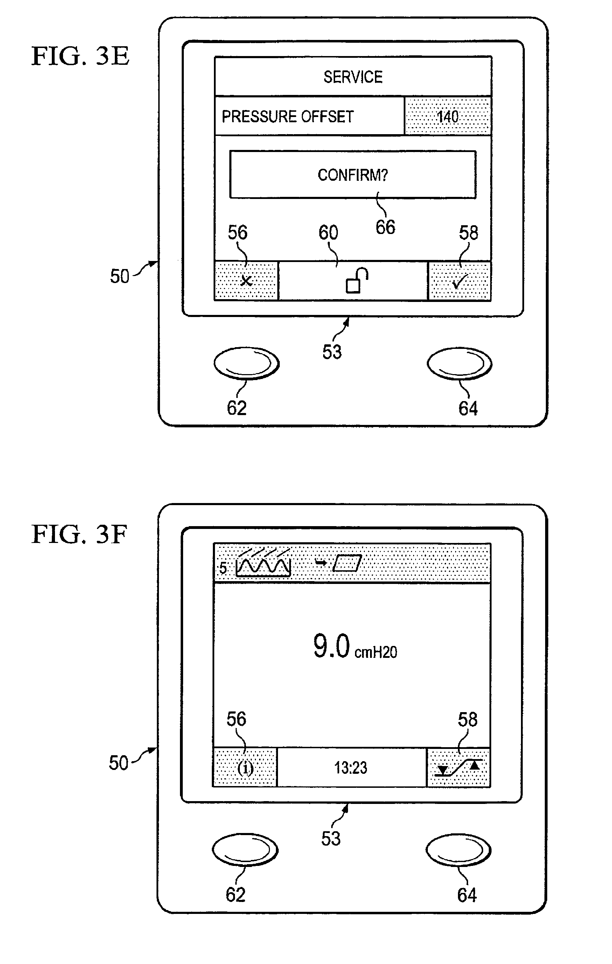

As yet another example, FIGURE 3E depicts an example screen that may be displayed to display 53 in the event a user is prompted to input a value using button 62 and/or button 64. As shown in FIGURE 3E, display 53 has displayed "CONFIRM?" to prompt a user to confirm a particular setting and/or parameter. Accordingly, the user's response to the prompt may have one of two values (e.g., "no" or "false" if the user desires not to confirm, or "yes" or "true" if the user desires to confirm). Thus, when such a prompt is displayed, function indicators 56 and 58 may indicate that their associated buttons 62 and/or 64 may be functional to select a response to the prompt. For example, function indicator 56 may display an "X" to indicate that button 62 is functional to select "no" or "false" and/or function indicator 58 may display a checkmark to indicate that button 64 is functional to select "yes" or "true" as shown in FIGURE 3E. In addition, modification indicator 60 may indicate that the displayed prompt may be responded to (e.g., modification indicator 60 may display an icon of an unlocked padlock as shown in FIGURE 3E, or display appropriate text such as "unlocked," for example).

As yet another example, FIGURE 3F depicts an example screen that may be displayed to display 53 during operation of breathing assistance system 10. During such operation, a user may press buttons 62 and/or 64 in order to access information menus regarding breathing assistance system 10 and/or access parameter menus allowing the user to modify operational parameters of breathing assistance system 10. Accordingly, function indicators 56 and 58 may display a graphic indicating the functionality of associated buttons 62 and 64. For example, function indicator 56 may display a graphic to indicate that button 62 is functional to access an information menu, and/or function indicator 58 may display a graphic to indicate that button 64 is functional to access a parameter menu as shown in FIGURE 3F.

Using the methods and systems disclosed herein, problems associated conventional approaches to breathing assistance system user interfaces may be reduced or eliminated. For example, because the methods and systems disclosed may allow for the functionality of user input buttons to change depending on the context of a display, allow for an indication of such functionality, and allow for an indication of whether parameters displayed may be modified, a user interface may be designed with fewer input buttons (allowing for a compact space-saving design) while providing an intuitive user experience.

Although the disclosed embodiments have been described in detail, it should be understood that various changes, substitutions and alterations can be made.

## Claims

1. A breathing assistance system user interface, comprising:
a plurality of user input buttons (62, 64) configured to receive user input; and
a display (53) communicatively coupled to the plurality of buttons (62, 64) and including:
a plurality of function indicators (56, 58), each function indicator associated with a corresponding one of the user input buttons (62, 64) and configured to dynamically display different graphical function representations indicating different functions of the corresponding user input button (62, 64) in different situations;
**characterized in that**:
the plurality of buttons (62, 64) are separate from the display (53); and
the display further comprises a modification indicator (60) configured to display different graphical modification representations in different situations to indicate whether or not a selected parameter value (55) related to operation of the breathing assistance system may be modified using one or more of the user input buttons (62, 64).

2. A user interface according to Claim 1,
wherein one of the graphical function representations indicates that the user input button (62, 64) associated with the function indicator (56, 58) is functional to increase the selected parameter value (55).

3. A user interface according to Claim 1,
wherein one of the graphical function representations indicates that the button (62, 64) associated with the function indicator (56, 58) is functional to decrease the selected parameter value (55).

4. A user interface according to Claim 1,
wherein one of the graphical function representations indicates that the button (62, 64) associated with the function indicator (56, 58) is not functional.

5. A user interface according to Claim 1,
wherein one of the graphical function representations indicates that the button (62, 64) associated with the function indicator (56, 58) is functional to select the parameter value.

6. A user interface according to Claim 1,
wherein one of the graphical function representations indicates that the button (62, 64) associated with the function indicator (56, 58) is functional to respond to a prompt displayed on the display (53).

7. A user interface according to Claim 1,
wherein one of the graphical function representations indicates that the button (62, 64) associated with the function indicator (56, 58) is functional to access a menu.

8. A breathing assistance system (10) comprising:
a gas delivery system (20) operable to supply breathable gas;
a patient interface (24) configured to interface with a patient for delivering breathable gas to the patient;
a connection system (22) configured to communicate breathable gas supplied by the gas delivery system to the patient interface for delivery to the patient;
a user interface (50) according to any preceding claim, the user interface (50) further including a control module (44) communicatively coupled to the user interface (50) and configured to regulate the delivery of breathable gas to the patient based at least on user input received by the user interface (50).

9. A method for displaying user interface functionality information for a breathing assistance system to a user, including:
providing a plurality of user input buttons (62, 64) configured to receive user input;
displaying a plurality of function indicators (56, 58) on the display (53), each function indicator associated with a corresponding one of the user input buttons (62, 64) and configured to dynamically display different graphical function representations indicating different functions of the corresponding user input button (62, 64) in different situations;
**characterized in that** the plurality of buttons (62, 64) are separate from a display (53),
wherein the method further comprises displaying a modification indicator (60) configured to display different graphical modification representations in different situations to indicate whether or not a selected parameter value (55) may be modified using one or more of the user input buttons (62, 64).

10. A method according to Claim 9,
wherein one of the graphical function representations (56, 58) indicates that the user input button (62, 64) associated with the function indicator (56, 58) is functional to increase the selected parameter value.

11. A method according to Claim 9,
wherein one of the graphical function representations indicates that the button (62, 64) associated with the function indicator (56, 58) is functional to decrease the selected parameter value (55).

12. A method according to Claim 9,
wherein one of the graphical function representations indicates that the button (62, 64) associated with the function indicator (56, 58) is not functional.

13. A method according to Claim 9,
wherein one of the graphical function representations indicates that the button (62, 64) associated with the function indicator (56, 58) is functional to select the parameter value (55).

14. A method according to Claim 9,
wherein one of the graphical function representations indicates that the button (62, 64) associated with the function indicator (56, 58) is functional to respond to a prompt displayed on the display (53).

15. A method according to Claim 9,
wherein one of the graphical function representations indicates that the button (62, 64) associated with the function indicator (56, 58) is functional to access a menu.

## Patentansprüche

1. Benutzerschnittstelle eines Atemhilfssystems, mit:
mehreren Benutzereingabetasten (62, 64), die zum Empfangen einer Benutzereingabe konfiguriert sind, und
einem Display (53), das datentechnisch mit den mehreren Tasten (62, 64) verbunden ist und aufweist:
mehrere Funktionsindikatoren (56, 58), wobei jeder Funktionsindikator einer entsprechenden der Benutzereingabetasten (62, 64) zugehörig und dazu konfiguriert ist, dynamisch unterschiedliche graphische Funktionsdarstellungen anzuzeigen, die verschiedene Funktionen der entsprechenden Benutzereingabetaste (62, 64) in verschiedenen Situationen angeben,
**dadurch gekennzeichnet, dass**
die mehreren Tasten (62, 64) von dem Display (53) getrennt sind, und
das Display ferner einen Modifikationsindikator (60) umfasst, der dazu konfiguriert ist, verschiedene graphische Modifikationsdarstellungen in verschiedenen Situationen anzuzeigen, um anzugeben, ob ein einen Betrieb des Atemhilfssystems betreffender, ausgewählter Parameterwert (55) mittels einer oder mehrerer der Benutzereingabetasten (62, 64) verändert werden kann oder nicht.

2. Benutzerschnittstelle nach Anspruch 1,
bei der eine der graphischen Funktionsdarstellungen angibt, dass die dem Funktionsindikator (56, 58) zugehörige Benutzereingabetaste (62, 64) dazu funktionsfähig ist, den ausgewählten Parameterwert (55) zu erhöhen.

3. Benutzerschnittstelle nach Anspruch 1,
bei der eine der graphischen Funktionsdarstellungen angibt, dass die dem Funktionsindikator (56, 58) zugehörige Taste (62, 64) dazu funktionsfähig ist, den ausgewählten Parameterwert (55) zu erniedrigen.

4. Benutzerschnittstelle nach Anspruch 1,
bei der eine der graphischen Funktionsdarstellungen angibt, dass die dem Funktionsindikator (56, 58) zugehörige Taste (62, 64) keine Funktion hat.

5. Benutzerschnittstelle nach Anspruch 1,
bei der eine der graphischen Funktionsdarstellungen angibt, dass die dem Funktionsindikator (56, 58) zugehörige Taste (62, 64) dazu funktionsfähig ist, den Parameterwert auszuwählen.

6. Benutzerschnittstelle nach Anspruch 1,
bei der eine der graphischen Funktionsdarstellungen angibt, dass die dem Funktionsindikator (56, 58) zugehörige Taste (62, 64) dazu funktionsfähig ist, auf eine auf dem Display (53) angezeigte Aufforderung zu reagieren.

7. Benutzerschnittstelle nach Anspruch 1,
bei der eine der graphischen Funktionsdarstellungen angibt, dass die dem Funktionsindikator (56, 58) zugehörige Taste (62, 64) dazu funktionsfähig ist, auf ein Menü zuzugreifen.

8. Atemhilfssystem (10), mit:
einem Gaszufuhrsystem (20), das dazu betriebsfähig ist, atembares Gas zu liefern,
einer Patientenschnittstelle (24), die dazu konfiguriert ist, mit einem Patienten zur Abgabe von atembarem Gas an den Patienten zu koppeln,
einem Verbindungssystem (22), das dazu konfiguriert ist, von dem Gaszufuhrsystem geliefertes, atembares Gas an die Patientenschnittstelle zur Abgabe an den Patienten zu leiten,
einer Benutzerschnittstelle (50) nach einem der vorhergehenden Ansprüche, wobei die Benutzerschnittstelle (50) ferner ein Steuerungsmodul (44) umfasst, das datentechnisch mit der Benutzerschnittstelle (50) verbunden und dazu konfiguriert ist, die Abgabe atembaren Gases an den Patienten basierend auf wenigstens einer mittels der Benutzerschnittstelle (50) empfangenen Benutzereingabe zu regeln.

9. Verfahren zum Anzeigen von Benutzerschnittstellenfunktionalitätsinformation für ein Atemhilfssystem an einen Benutzer, umfassend:
Bereitstellen mehrerer Benutzereingabetasten (62, 64), die dazu konfiguriert sind, eine Benutzereingabe zu empfangen,
Anzeigen mehrerer Funktionsindikatoren (56, 58) auf dem Display (53), wobei jeder Funktionsindikator einer entsprechenden der Benutzereingabetasten (62, 64) zugehörig und dazu konfiguriert ist, dynamisch verschiedene graphische Funktionsdarstellungen anzuzeigen, die verschiedene Funktionen der entsprechenden Benutzereingabetaste (62, 64) in verschiedenen Situationen angeben,
**dadurch gekennzeichnet, dass** die mehreren Tasten (62, 64) von einem Display (53) getrennt sind,
wobei das Verfahren ferner umfasst ein Anzeigen eines Modifikationsindikators (60), der dazu konfiguriert ist, verschiedene graphische Modifikationsdarstellungen in verschiedenen Situationen anzuzeigen, um anzugeben, ob ein ausgewählter Parameterwert (55) mittels einer oder mehrerer der Benutzereingabetasten (62, 64) geändert werden kann oder nicht.

10. Verfahren nach Anspruch 9,
bei dem eine der graphischen Funktionsdarstellungen angibt, dass die dem Funktionsindikator (56, 58) zugehörige Benutzereingabetaste (62, 64) dazu funktionsfähig ist, den ausgewählten Parameterwert (55) zu erhöhen.

11. Verfahren nach Anspruch 9,
bei dem eine der graphischen Funktionsdarstellungen angibt, dass die dem Funktionsindikator (56, 58) zugehörige Taste (62, 64) dazu funktionsfähig ist, den ausgewählten Parameterwert (55) zu erniedrigen.

12. Verfahren nach Anspruch 9,
bei dem eine der graphischen Funktionsdarstellungen angibt, dass die dem Funktionsindikator (56, 58) zugehörige Taste (62, 64) keine Funktion hat.

13. Verfahren nach Anspruch 9,
bei dem eine der graphischen Funktionsdarstellungen angibt, dass die dem Funktionsindikator (56, 58) zugehörige Taste (62, 64) dazu funktionsfähig ist, den Parameterwert auszuwählen.

14. Verfahren nach Anspruch 9,
bei dem eine der graphischen Funktionsdarstellungen angibt, dass die dem Funktionsindikator (56, 58) zugehörige Taste (62, 64) dazu funktionsfähig ist, auf eine auf dem Display (53) angezeigte Aufforderung zu reagieren.

15. Verfahren nach Anspruch 9,
bei dem eine der graphischen Funktionsdarstellungen angibt, dass die dem Funktionsindikator (56, 58) zugehörige Taste (62, 64) dazu funktionsfähig ist, auf ein Menü zuzugreifen.

## Revendications

1. Interface utilisateur pour un système d'assistance respiratoire, comprenant :
une pluralité de boutons d'entrée utilisateur (62, 64) configurés pour recevoir une entrée utilisateur ; et
un affichage (53) couplé en communication à la pluralité de boutons (102, 64) et comprenant :
une pluralité d'indicateurs de fonctions (56, 58), chaque indicateur de fonctions étant associé à l'un correspondant parmi les boutons d'entrée utilisateur (62, 64) et configuré pour afficher de façon dynamique différentes représentations de fonctions graphiques indiquant différentes fonctions des boutons d'entrée utilisateur correspondants (62, 64) dans différentes situations ; **caractérisée en ce que** :
la pluralité de boutons (62, 64) sont séparés de l'affichage (53) ; et
l'affichage comprend en outre un indicateur de modification (60) configuré pour afficher différentes représentations de modifications graphiques dans différentes situations pour indiquer si oui ou non une valeur de paramètre sélectionnée (55) se rapportant à une opération du système d'assistance respiratoire peut être modifiée en utilisant un ou plusieurs parmi les boutons d'entrée utilisateur (60, 64).

2. Interface utilisateur selon la revendication 1,
dans laquelle l'une des représentations de fonctions graphiques indique que le bouton d'entrée utilisateur (62, 64) associé à l'indicateur de fonctions (56, 58) est fonctionnel pour augmenter la valeur de paramètre sélectionnée (55).

3. Interface utilisateur selon la revendication 1,
dans laquelle l'une des représentations de fonctions graphiques indique que le bouton (62, 64) associé à l'indicateur de fonctions (56, 58) est fonctionnel pour diminuer la valeur de paramètre sélectionnée (55).

4. Interface utilisateur selon la revendication 1,
dans laquelle l'une des représentations de fonctions graphiques indique que le bouton (62, 64) associé à l'indicateur de fonctions (56, 58) n'est pas fonctionnel.

5. Interface utilisateur selon la revendication 1,
dans laquelle l'une des représentations de fonctions graphiques indique que le bouton (62, 64) associé à l'indicateur de fonctions (56, 58) est fonctionnel pour sélectionner la valeur de paramètre.

6. Interface utilisateur selon la revendication 1,
dans laquelle l'une des représentations de fonctions graphiques indique que le bouton (62, 64) associé à l'indicateur de fonctions (56, 58) est fonctionnel pour répondre à un message affiché sur l'affichage (53).

7. Interface utilisateur selon la revendication 1,
dans laquelle l'une des représentations de fonctions graphiques indique que le bouton (62, 64) associé à l'indicateur de fonctions (56, 58) est fonctionnel pour accéder au menu.

8. Système d'assistance respiratoire (10) comprenant :
un système d'alimentation en gaz (20) utilisable pour délivrer un gaz respirable ;
une interface patient (24) configurée pour communiquer avec un patient pour délivrer un gaz respirable au patient ;
un système de raccordement (22) configuré pour faire communiquer le gaz respirable délivré par le système d'alimentation en gaz avec l'interface patient pour alimenter le patient ;
une interface utilisateur (50) selon l'une quelconque des revendications précédentes, l'interface utilisateur (50) comprenant en outre un module de commande (44) couplé en communication avec l'interface utilisateur (50) et configuré pour réguler l'alimentation en gaz respirable vers le patient sur la base au moins d'une entrée utilisateur reçue par l'interface utilisateur (50).

9. Procédé d'affichage d'informations de fonctionnalité de l'interface utilisateur pour un système d'assistance respiratoire à un utilisateur, comprenant les états consistant à :
prévoir une pluralité de boutons d'entrée utilisateur (62, 64) configurés pour recevoir une entrée utilisateur ;
afficher une pluralité d'indicateurs de fonctions (56, 58) sur l'affichage (53), chaque indicateur de fonctions étant associé à l'un correspondant parmi les boutons d'entrée utilisateur (62, 64) et configuré pour afficher de façon dynamique différentes représentations de fonctions graphiques indiquant différentes fonctions du bouton d'entrée utilisateur correspondant (62, 64) dans différentes situations ;
**caractérisé en ce que** la pluralité de boutons (62, 64) sont séparés de l'affichage (53),
dans lequel le procédé comprend en outre l'étape consistant à afficher un indicateur de modification (60) configuré pour afficher différentes représentations de modifications graphiques dans différentes situations pour indiquer si oui ou non une valeur de paramètre sélectionnée (55) peut être modifiée en utilisant l'un ou plusieurs parmi les boutons d'entrée utilisateur (62, 64).

10. Procédé selon la revendication 9,
dans lequel l'une des représentations de fonctions graphiques (56, 58) indique que le bouton d'entrée utilisateur (62, 64) associé à l'indicateur de fonctions (56, 58) est fonctionnel pour augmenter la valeur de paramètre sélectionnée.

11. Procédé selon la revendication 9,
dans lequel l'une des représentations de fonctions graphiques indique que le bouton (62, 64) associé à l'indicateur de fonctions (56, 58) est fonctionnel pour diminuer la valeur de paramètre sélectionnée (55).

12. Procédé selon la revendication 9,
dans lequel l'une des représentations de fonctions graphiques indique que le bouton (62, 64) associé à l'indicateur de fonctions (56, 58) n'est pas fonctionnel.

13. Procédé selon la revendication 9,
dans lequel l'une des représentations de fonctions graphiques indique que le bouton (62, 64) associé à l'indicateur de fonctions (56, 58) est fonctionnel pour sélectionner la valeur de paramètre sélectionnée (55).

14. Procédé selon la revendication 9,
dans lequel l'une des représentations de fonctions graphiques indique que le bouton (62, 64) associé à l'indicateur de fonctions (56, 58) est fonctionnel pour répondre à un message affiché sur l'affichage (53).

15. Procédé selon la revendication 9,
dans lequel l'une des représentations de fonctions graphiques indique que le bouton (62, 64) associé à l'indicateur de fonctions (56, 58) est fonctionnel pour accéder au menu.
